# EUROPÄISCHE PATENTSCHRIFT

(11) **EP 2 830 567 B1**
(45) Veröffentlichungstag und Bekanntmachung des Hinweises auf die Patenterteilung: **30.12.2015**
(21) Anmeldenummer: 13713786.5
(22) Anmeldetag: 20.03.2013
(51) Int. Cl.: A61G 11/00

(54) **WÄRMETHERAPIEGERÄT**
THERMOTHERAPY DEVICE
APPAREIL DE THERMOTHÉRAPIE

(30) Priorität: 27.03.2012 DE 102012006204
(43) Veröffentlichungstag der Anmeldung: 04.02.2015
(73) Patentinhaber: Drägerwerk AG & Co. KGaA, 23558 Lübeck (DE)
(72) Erfinder: BOHNEN, Thomas, 23568 Lübeck (DE); GERBRANDA, Tjeerd Jan Pieter, NL-2565 GJ Den Haag (NL); HAMPE, Markus, 23562 Lübeck (DE); KOCH, Jochim, 23909 Ratzeburg (DE); MÖHRING, Philip, 23558 Lübeck (DE)
(74) Vertreter: Kettenbeil, Roxane Henriette
(86) Internationale Anmeldenummer: PCT/EP2013/055845
(87) Internationale Veröffentlichungsnummer: WO 2013/143949

(56) Entgegenhaltungen:
- EP-A1- 2 168 545
- EP-A2- 2 179 718
- DE-A1- 2 913 282
- DE-A1-102010 014 561

## Beschreibung

Die vorliegende Erfindung betrifft ein Wärmetherapiegerät, insbesondere Inkubator, zur Behandlung von Neugeborenen mit einer umrandeten, von oben frei zugänglichen Liegefläche zur Aufnahme eines Neugeborenen, einer Haube, die zwischen einer die Liegefläche abdeckenden geschlossenen Stellung und einer die Liegefläche freigebenden offenen Stellung beweglich ist, und einer an einer Tragstruktur aufgehängten, auf die Liegefläche gerichteten Strahlungsheizung.

Ein derartiges Wärmetherapiegerät ist beispielsweise aus DE 20 2005 021 580 U1 bekannt. Das bekannte Wärmetherapiegerät hat eine an einer Tragstruktur vertikal beweglich aufgehängte Haube. Ferner ist an der Tragstruktur eine Strahlungsheizung angebracht, die auf die Liegefläche gerichtet ist. In der geöffneten Stellung der Haube liegt diese vertikal noch unterhalb der Strahlungsheizung. Die Haube ist teilweise für IR-Strahlung transparent, so dass von der Strahlungsheizung auf die Liegefläche gerichtete Wärmestrahlung IR-transparente Teile der Haube durchstrahlen und so die Liegefläche erreichen kann. Mit diesem Gerät kann eine kontinuierliche Wärmezufuhr realisiert werden, d.h. die Strahlungsheizung kann ohne Unterbrechung arbeiten, während die Haube aus der geschlossenen Stellung in die geöffnete Stellung oder umgekehrt bewegt wird. Allerdings wird die Wärmestrahlung von der Strahlungsheizung beim Durchgang durch die teilweise IR-transparente Haube gleichwohl abgeschwächt. Das Material für die IR-transparenten Bereiche ist zum einen sehr teuer und zum anderen sind die IR-transparenten Bereiche nur schwer in die Haube zu integrieren.

Aus US 6,231,499 B1 ist ein weiteres Wärmetherapiegerät bekannt. Hier wird die Haube über einen elektrischen linearen Antrieb nach oben gefahren, wodurch der Öffnungsvorgang relativ lange dauert. Die Strahlungsheizung ist innerhalb der Haube integriert. Sie schaltet sich aus Sicherheitsgründen erst ein, wenn die Haube vollständig hochgefahren ist, und muss beim Herunterfahren der Haube sofort wieder ausgeschaltet und durch einen Klappdeckel verdeckt werden, um das Baby vor dem zu diesem Zeitpunkt noch sehr heißen Strahler zu schützen. Eine gleichzeitige Beheizung der Haube im geschlossenen Zustand, beispielsweise zur Vermeidung von Kondensation von Wasserdampf an der Innenwand der Haube und zur Vermeidung von Strahlungsverlusten des Säuglings, ist nicht möglich.

Aus US 7 282 022 ist ein Wärmetherapiegerät mit feststehendem Heizstrahler und mit einer Klappe in der Haube bekannt, die geöffnet werden muss, um das Kind bei offen stehender Haube ungehindert bestrahlen zu können. Dies bedingt eine aufwändige Mechanik. Außerdem erreicht die volle Wärmestrahlung das Kleinkind erst bei oben stehender Haube und offen stehender Klappe.

Ein weiteres Wärmetherapiegerät ist US 5,971,914 B1 bekannt. Hier werden zwei in der Länge getrennte Haubenteile von der Strahlungsheizung getragen, wobei sich beim Öffnen die beiden Haubenteile nach oben falten. Die Strahlungsheizung kann hochgefahren und für Röntgenuntersuchungen zur Seite geschwenkt werden. Die Stellung der Haube und die Position der Strahlungsheizung beeinträchtigen in geöffnetem Zustand den Zugang zum Patienten.

Aus WO 2009/073693 ist ein Wärmetherapiegerät bekannt, bei dem eine nach hinten wegschwenkbare Haube um eine am Kopfende des Wärmetherapiegerätes liegende Schwenkachse nach hinten schwenkbar ist. Durch das Schwenken der Haube hinter das Kopfende des Patienten muss die Strahlungsheizung in einer erheblichen Höhe über der Liegefläche angeordnet sein, was sich auf die mechanische Stabilität und die Transportfähigkeit des Wärmetherapiegerätes auswirkt.

Weitere Wärmetherapiegeräte sind aus EP 2 179 718 A2 und DE 10 2010 014 561 A1 bekannt.

Es ist Aufgabe der vorliegenden Erfindung, ein verbessertes Wärmetherapiegerät anzugeben, das einen Betrieb der Strahlungsheizung sowohl in der geschlossenen als auch in der geöffneten Stellung der Haube ermöglicht.

Zur Lösung dieser Aufgabe dienen die kennzeichnenden Merkmale des Patentanspruchs 1 in Verbindung mit dessen Oberbegriff. Vorteilhafte Ausführungsformen der Erfindung sind in den Unteransprüchen aufgeführt.

Erfindungsgemäß ist vorgesehen, dass die Haube an der Tragstruktur so aufgehängt ist, dass sie nach der Bewegung aus der geschlossenen in die geöffnete Stellung mit ihrem der Tragstruktur zugewandten Ende vertikal wenigstens auf Höhe der Strahlungsheizung und horizontal weiter von der Tragstruktur entfernt als diese liegt, so dass die Haube eine Position außerhalb des Strahlungskegels von der Strahlungsheizung auf die Liegefläche einnimmt. Die Haube wird so aus dem Strahlengang von der Strahlungsheizung zur Liegefläche gebracht, wodurch weder eine Klappe in der Haube noch IR-transparente Materialien benötigt werden.

Vorzugsweise ist die Aufhängung der Haube an der Tragstruktur so ausgestaltet, dass in der geöffneten Stellung der Haube deren von der Tragstruktur abgewandtes Ende höher als das ihr zugewandte Ende liegt. Dadurch wird die Zugänglichkeit der Liegefläche verbessert.

Das der Tragstruktur zugewandte Ende der Haube wird durch einen Kopplungsmechanismus auf einer Kreisbahn um einen Drehpunkt an der Befestigung des Kopplungsmechanismus an der Tragstruktur geführt. Die Haube kann so durch eine einfache, schnelle Bewegung von der geschlossenen in die geöffnete Stellung und umgekehrt gebracht werden.

Vorzugsweise kann der Kopplungsmechanismus zwischen Tragstruktur und Haube als viergliedriges Getriebe mit zwei Schwingen (Gelenkviereck) ausgeführt sein. Alternativ kann der Kopplungsmechanismus zwischen Tragstruktur und Haube als überbestimmtes fünfgliedriges Getriebe mit drei Schwingen ausgeführt sein.

Alternativ kann die Haube durch einen Kopplungsmechanismus schwenkbar an der Tragstruktur aufgehängt sein, der dadurch gekennzeichnet ist, dass eine mit der Bewegung der Haube aus der geschlossenen in die geöffnete Stellung einhergehende Winkel-Positionierung der Haube relativ zur Liegefläche durch einen an eine Schwinge gekoppelten Kettentrieb oder einen formschlüssigen Zahnriementrieb mit einem fest mit der Haube verbundenen Kettenrad beziehungsweise Zahnriemenscheibe und einem fest mit der Tragstruktur verbundenen Kettenrad beziehungsweise Zahnriemenscheibe bewirkt wird. Vorzugsweise ist dann eine Vorrichtung zum Spannen des Ketten- oder Zahnriementriebes vorgesehen.

Vorzugsweise ist die Tragstruktur als Zentralsäule ausgebildet und ist der Kopplungsmechanismus zwischen Tragstruktur und Haube nur einseitig an der Tragstruktur montiert. An der dem Kopplungsmechanismus gegenüberliegenden Seite sind Bedienkomponenten, die in die Zentralsäule eingebaut sind (z. B. Anzeige- und Bedieneinheit, Notfallbeatmungseinheit) dann frei zugänglich.

Vorzugsweise kann eine der Schwingen oder die Schwinge als ein allseitig geschlossenes Gehäuse ausgebildet sein, das die anderen Komponenten des Getriebes umschließt.

Vorzugsweise sind Mittel zum manuellen Öffnen und Schließen der Haube oder ist ein Antrieb zum Öffnen und Schließen der Haube vorgesehen. Zum manuellen Öffnen kann ein Griff in dem der Tragstruktur zugewandten Bereich der Haube beidseitig am Haubenrand angeordnet sein.

Alternativ oder zusätzlich ist zum Bewirken einer Drehbewegung ein Aktuator oder ein Motor vorgesehen, der an dem Drehpunkt zwischen Kopplungsmechanismus und Tragstruktur ansetzt.

Vorzugsweise sind elektrische Endstellungssensoren vorhanden, die die geöffnete und geschlossene Stellung der Haube detektieren. Beispielsweise sind die Endstellungssensoren als Taster, Hallsensoren oder als Lichtschranken ausgeführt.

In einer bevorzugten Ausführungsform ist der Kopplungsmechanismus mit einem Feder- und/oder Dämpfer-Mechanismus ausgestattet.

Von Vorteil ist, wenn der Feder- und/oder Dämpfermechanismus derart ausgestaltet ist, dass die Haube in die geschlossene Stellung bewegt wird, solange die Haube weniger als einen Schwellenwert aus der geschlossenen Stellung herausgeschwenkt ist, und sie in die vollständig geöffnete Stellung bewegt wird, sobald die Haube um mehr als den Schwellenwert aus der geschlossenen Stellung herausgeschwenkt ist.

Vorzugsweise ist der Feder- und/oder Dämpfermechanismus in die als Gehäuse gestaltete Schwinge integriert und wird von dieser umschlossen.

Vorzugsweise ist der Feder- und/oder Dämpfermechanismus zwischen mindestens einer Schwinge und der Tragstruktur und/oder zwischen zwei Schwingen angeordnet. Dabei kann der Feder- und/oder Dämpfermechanismus eine Gasdruckfeder aufweisen. Vorzugsweise kann die Positionierung der Haube über eine einen Bolzen umschließende Schelle in axialer Richtung des Bolzens als auch in ihrer Winkellage relativ zur Liegefläche einstellbar sein.

Die Erfindung wird im Folgenden anhand von Ausführungsbeispielen in den Zeichnungen beschrieben, in denen
Figur 1 eine schematische Seitenansicht eines Wärmetherapiegeräts mit der Haube in der geschlossenen Stellung in durchgezogenen Linien und der Haube in der geöffneten Stellung in durchbrochenen Linien zeigt,
Figur 2 Teil-Seitenansichten eines Wärmetherapiegeräts in geschlossener und geöffneter Stellung zeigt, wobei ein Ausführungsbeispiel des Kopplungsmechanismus aus Figur 1 dargestellt ist,
Figur 3 eine Explosionsansicht des Kopplungsmechanismus aus Figur 2 zeigt,
Figur 4 eine Teil-Seitenansicht eines Wärmetherapiegerätes mit einem Hubarm mit einem Zahnriemen- oder Kettentrieb zeigt,
Figur 5 eine schematische Teil-Seitenansicht eines motorischen Antriebs und von elektrischen Tastern zur Detektierung der Endstellungen zeigt, und
Figur 6 eine schematische Ansicht auf ein Wärmetherapiegerät von oben mit justierbarer Haubenaufhängung zeigt.

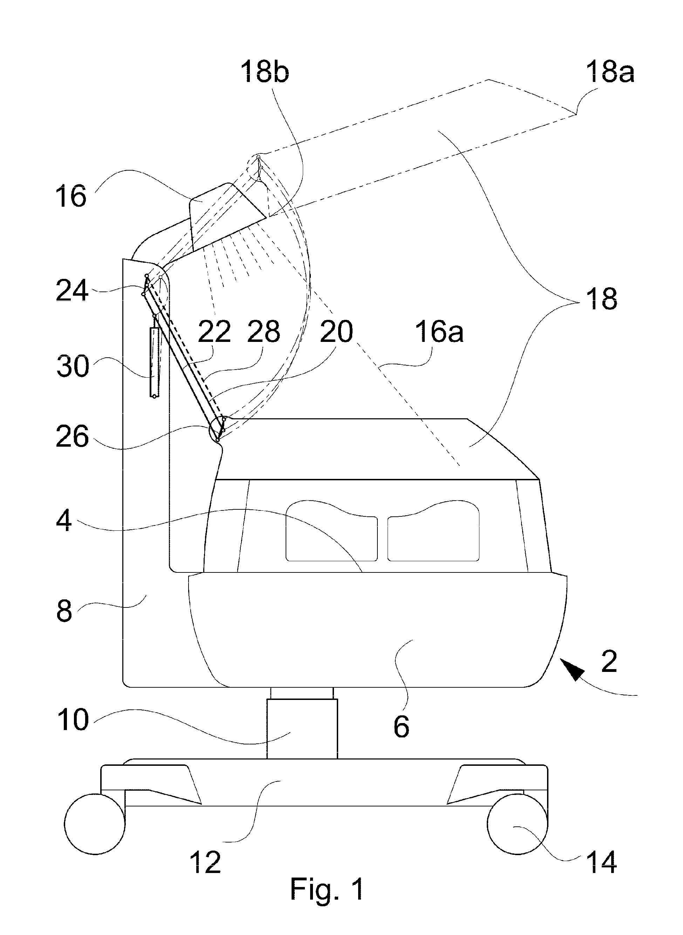

Das Wärmetherapiegerät 2 in Fig. 1 hat eine umrandete Liegefläche 4, die ein Neugeborenes auf der Liegefläche liegend aufnehmen kann. Die Liegefläche 4 wird von einer horizontalen Tragstruktur 6 getragen, an der wiederum eine vertikale Tragstruktur 8 befestigt ist. Die horizontale Tragstruktur 6 ist über eine Hubsäule 10 mit einer horizontalen Basis 12 verbunden, die wiederum mit Rollen 14 zum Transport des Wärmetherapiegerätes versehen ist.

An der Tragstruktur 8, die sich am Kopfende der umrandeten Liegefläche 4 befindet, ist oben eine Strahlungsheizung 16 aufgehängt, die so ausgerichtet ist, dass ihr Strahlungskegel auf die umrandete Liegefläche 4 fällt. In der Fig. 1 ist die geöffnete Stellung der Haube 18 in durchbrochenen Linien dargestellt, während die Haube 18 in geschlossener Stellung mit durchgezogenen Linien gezeigt ist.

Der Kopplungsmechanismus der Haube 18 zur Tragstruktur 8 ist dabei als Viergelenk mit zwei Schwingen 20, 22 so ausgestaltet, dass beim Schwenken aus der mit durchgezogenen Linien gezeigten geschlossenen Stellung der Haube 18 in die mit durchbrochenen Linien gezeigte geöffnete Stellung die Haube 18 in eine Schrägstellung gebracht wird, so dass ihr von der Tragstruktur 8 entfernt liegendes Ende 18a vertikal höher als das der Tragstruktur 8 zugewandte Ende 18b liegt. Das Getriebeglied 24 ist fest mit der Tragstruktur 8 verbunden und das Getriebeglied 26 ist fest mit der Haube 18 verbunden. Wären die Längen der Getriebeglieder 20 und 22 sowie die Längen der Getriebeglieder 24 und 26 jeweils gleich, so würden sie ein Parallelogrammgetriebe bilden und eine reine Verschiebung der Haube 18 bewirken. Tatsächlich sind die Gliedlängen der Getriebeglieder 20 und 22 bzw. 24 und 26 in der dargestellten Ausführungsform alle leicht unterschiedlich, um die beschriebene Schrägstellung der Haube 18 beim Öffnen zu realisieren. Diese erfindungsgemäße Gestaltung des Getriebes zur Haubenöffnung dient dazu, die Zugänglichkeit für das Pflegepersonal sowie für ein Röntgengerät oder dergleichen zu verbessern.

Eine optionale dritte Schwinge 28 führt zwar theoretisch zu einer statischen Überbestimmung des Getriebes, dient aber in der praktischen Ausführung der Versteifung, indem ungünstige Zustände des Mechanismus in der Nähe von Totpunkten vermieden werden. Der so erweiterte Getriebemechanismus ist dann anstatt als viergliedriger Mechanismus als fünfgliedriger Mechanismus aufzufassen.

Die Bewegung der Haube 18 aus der geschlossenen in die geöffnete Stellung und umgekehrt kann durch ein Feder-DämpferElement 30, beispielsweise eine Gasdruckfeder unterstützt werden, die beispielsweise zwischen der Tragstruktur 8 und dem Getriebeglied 22 angreift. Stattdessen könnte der Feder-Dämpfer-Mechanismus auch zwischen Tragstruktur 8 und Getriebeglied 20 oder 28 oder auch zwischen zweien der Getriebeglieder 20, 22 oder 28 angreifen. Auf diese Weise kann die Haube 18 mit einer wenig Kraft erfordernden Bewegung aus der geschlossenen in die geöffnete Stellung und umgekehrt bewegt werden, wobei der Federmechanismus gegenüber dem Eigengewicht der Haube 18 so ausgelegt ist, dass die Haube 18 beim Öffnen selbsttätig in die vollständig geöffnete Stellung gezogen wird, sobald ein vorgegebener Schwenkungsgrad überschritten ist, und beim Schließen selbsttätig vollständig geschlossen wird, sobald ein vorgegebener Schwenkungsgrad unterschritten ist.

Die Strahlungsheizung 16 ist oben an der Tragstruktur 8 angebracht und so ausgerichtet, dass die Haube 18 in ihrer geöffneten Stellung den Strahlengang 16a der Strahlungsheizung auf die Liegefläche 4 nicht behindert.

Auf diese Weise ist es insbesondere möglich, dass die Strahlungsheizung 16 während des geschlossenen Betriebs, also bei geschlossener Haube 18 arbeitet, um so, wenn erforderlich, die Haube 18 zu erwärmen, um Kondensationen an der Innenfläche der Haube 18 zu verhindern. Darüber hinaus ist es möglich, dass die Strahlungsheizung 16 bereits vor dem Öffnen der Haube 18 auf ihre Betriebstemperatur gebracht wird und die Haube erst dann geöffnet wird, wenn die Strahlungsheizung 16 sofort die gewünschte Heizleistung auf der Liegefläche erbringen kann. Im Stand der Technik, bei dem die Strahlungsheizung erst nach dem Öffnen der Haube einschaltbar ist, konnte eine Übergangszeit, in der die Strahlungsheizung auf ihre Betriebstemperatur kommt, verstreichen, in der der Patient nicht ausreichend mit Wärme versorgt wird. Dieses Problem wird durch die erfindungsgemäße Gestaltung überwunden, da die Strahlungsheizung (16) auf ihre Betriebstemperatur gebracht werden kann, bevor die Haube 18 geöffnet wird.

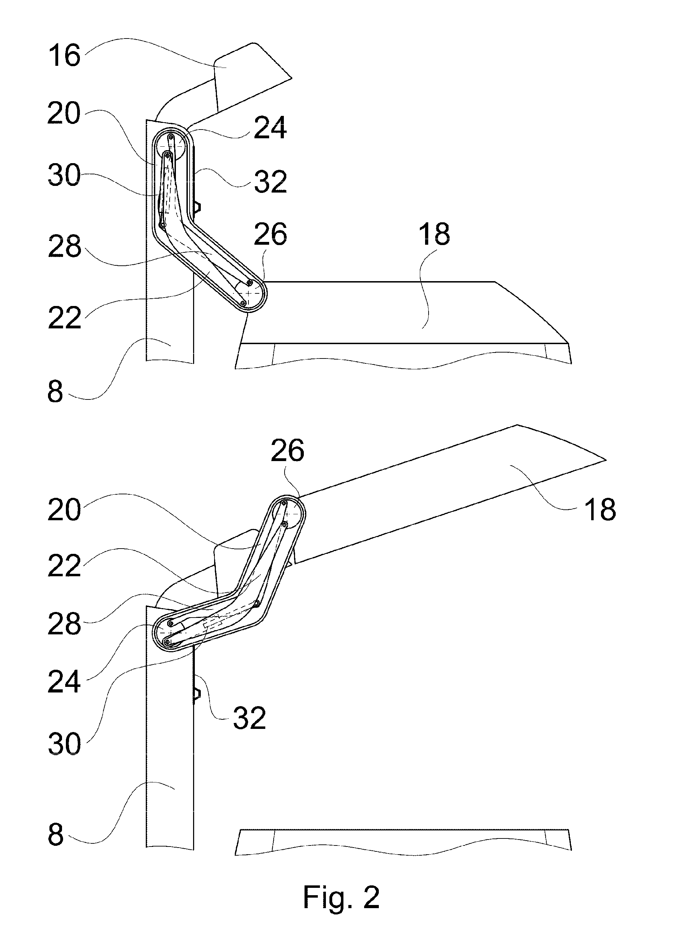

In Figur 2 ist eine Teilansicht eines Wärmetherapiegeräts mit der Haube 18 in geschlossener Stellung (oben) und in geöffneter Stellung (unten) gezeigt, die ein konstruktives Ausführungsbeispiel des in Fig. 1 beschriebenen Öffnungsmechanismus für die Haube 18 darstellt. Der Kopplungsmechanismus umfasst eine als Gehäuse ausgestaltete erste Schwinge 20 (im folgenden als Hubarm bezeichnet), eine zweite Schwinge 22 und eine dritte Schwinge 28, ein Verbindungsglied 24 zur Tragstruktur 8, ein Verbindungsglied 26 zur Haube 18 sowie eine Gasdruckfeder 30, die zwischen Verbindungsglied 24 und Schwinge 28 angreift. Beide Schwingen 22 und 28 sowie die Gasdruckfeder 30 sind in den Hubarm 20 eingebaut. Der Hubarm 20 sowie die in ihn eingebauten Schwingen 22 und 28 haben im Ausführungsbeispiel eine abgewinkelte Form, die bei geschlossener Haube teilweise parallel zur Tragstruktur 8 verläuft. Hierdurch wird zum Beispiel der Zugang zu einer in der Tragstruktur 8 zentral angeordneten Anzeige- und Bedieneinheit 32 erleichtert.

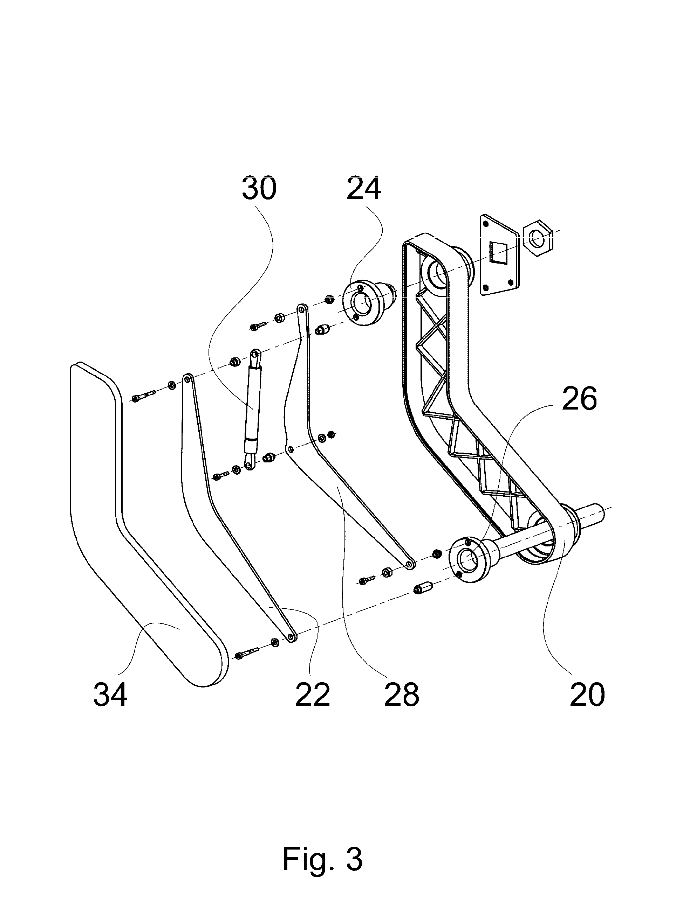

In Figur 3 ist eine Explosionsdarstellung des zuvor beschriebenen Ausführungsbeispiels des Kopplungsmechanismus dargestellt. Der Hubarm wird mit einem Deckel 34 verschlossen, so dass der Mechanismus komplett gekapselt ist, was die Verletzungsgefahr verringert und die Hygiene erleichtert.

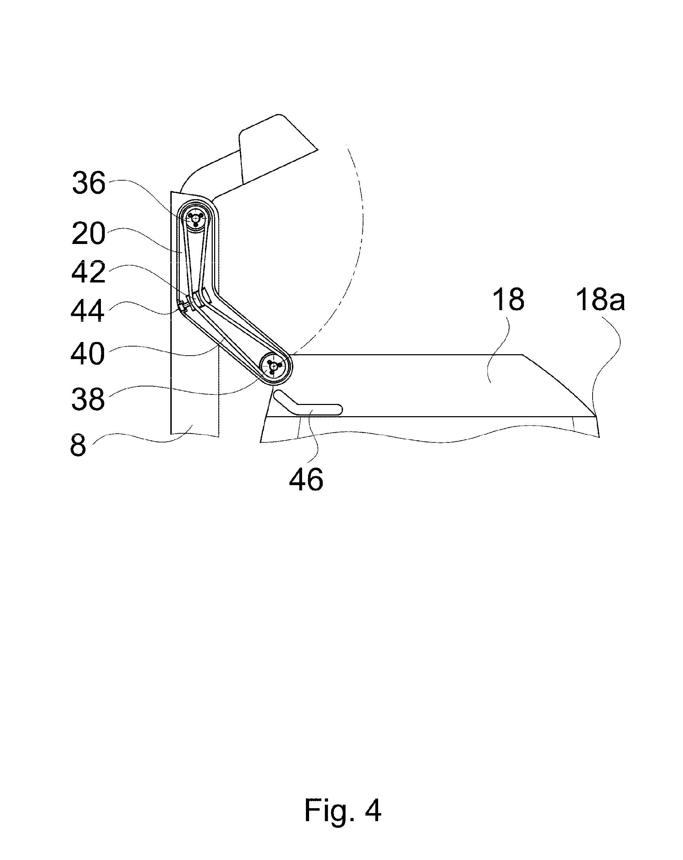

Eine alternative Ausführungsform des Kopplungsmechanismus der Haube ist in Figur 4 gezeigt. In dem ebenfalls als abgewinkeltes Gehäuse gestalteten Hubarm 20 ist ein Kettenrad oder eine Zahnriemenscheibe 36, im folgenden erstes Ritzel genannt, fest mit der Tragstruktur 8 verbunden. Das gegenüberliegende Kettenrad bzw. die Zahnriemenscheibe 38, im Folgenden zweites Ritzel genannt, ist fest mit der Haube 18 verbunden. Eine Kette bzw. ein Zahnriemen 40 ist um das erste und zweite Ritzel 36, 38 und durch eine Umlenkung 42 geführt, um den Mechanismus der Form des Hubarms 20 anzupassen. Die Umlenkung 42 kann auch zum Einstellen der Ketten-Vorspannung bzw. Zahnriemen-Vorspannung verwendet werden, z. B. über eine Spannschraube 44. Das starre Getriebeglied, hier der Hubarm 20, ist derart an den Kettentrieb bzw. Zahnriementrieb gekoppelt, dass es das zweite Ritzel 38 und damit das der Tragstruktur zugewandte Ende der Haube 18 auf einer Kreisbahn führt. Die Kette oder der Zahnriemen 40 läuft dabei über das erste Ritzel 36 ab und bestimmt durch die so auf das zweite Ritzel 38 übertragene Winkelbewegung die Lage der Haube 18 relativ zu einer horizontalen Ebene. Vorzugsweise ist das zweite Ritzel 38 etwas größer als das erste Ritzel 36, was beim Öffnen eine Schrägstellung der Haube 18 in derselben gewünschten Weise bewirkt wie in Fig. 1 und 2 gezeigt.

Bei einer manuellen Bedienung des Kopplungsmechanismus für die Haube 18 ist vorzugsweise beidseitig je ein Griff 46 an der Haube 18 montiert, der seitlich an dem der Tragstruktur 8 zugewandten Ende der Haube 18 angeordnet ist. So muss der Anwender beim Aufschwenken der Haube 18 nicht dem der Endstellung höher liegenden Ende an der von der Tragstruktur 8 abgewandten Seite der Haube 18a folgen. Ferner führt die Positionierung des Griffs an dem der Tragstruktur zugewandten Ende der Haube 18 auch zu einem günstig relativ zum Kopplungsmechanismus gelegenen Kraftangriffspunkt. Diese Griffanordnung ist genauso vorteilhaft bei einer Ausführung des Mechanismus nach Fig. 1 bis 3.

Eine Unterstützung der manuellen Bedienung durch einen in Figur 4 nicht dargestellten Feder-Dämpfer-Mechanismus, wie er zuvor beschrieben wurde, ist auch in diesem Ausführungsbeispiel sinnvoll, er kann zweckmäßig zwischen Tragstruktur 8 und Hubarm 20 angeordnet sein.

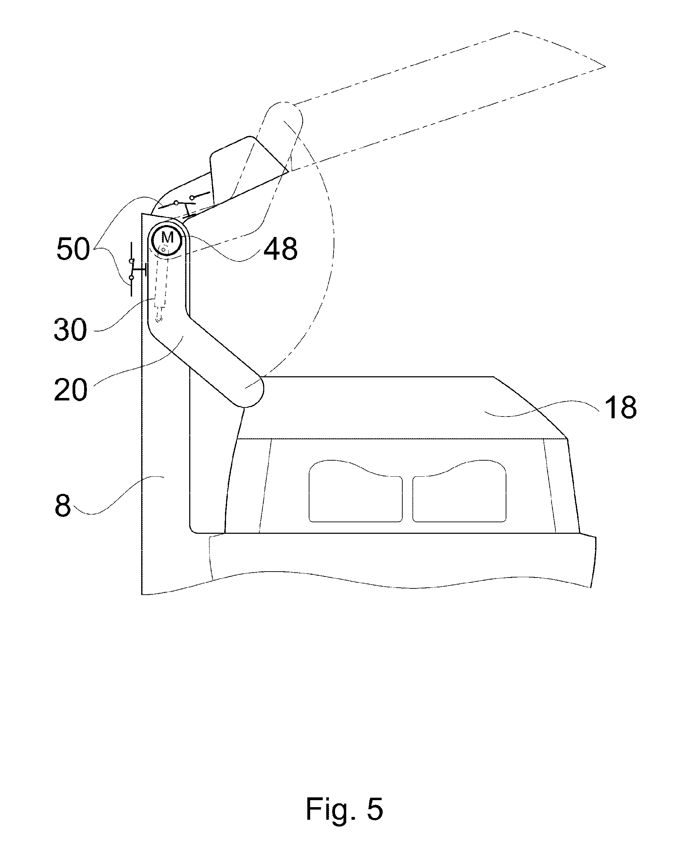

Ein alternatives Konzept, bei der die Haube nicht manuell, sondern über einen motorischen Antrieb geöffnet und geschlossen wird, zweigt schematisch Figur 5. Ein Motor 48 leitet sein Drehmoment vorzugsweise am mit der Tragstruktur 8 verbundenen Drehpunkt des Hubarms 20 ein. Um das Drehmoment klein zu halten, kann das Gewicht der Haube 18 auch hier z. B. durch einen Feder- und/oder Dämpfermechanismus, z. B. eine Gasdruckfeder 30 zumindest teilweise kompensiert werden. Hierbei kommt es überwiegend auf die Federwirkung an, da die Bewegungsgeschwindigkeit durch die Ansteuerung des Motors kontrolliert werden kann.

Sowohl bei motorischem wie bei manuellem Antrieb der Haubenöffnung muss die Steuerungssoftware des Gerätes Informationen erhalten, in welcher Stellung sich die Haube gegenwärtig befindet. Über zwei in diesem Ausführungsbeispiel als Taster ausgeführte Endstellungssensoren 50 lassen sich zumindest die drei Zustände offen, geschlossen und Zwischenstellung voneinander unterscheiden. Stattdessen oder zusätzlich kann eine Winkelmesseinrichtung vorgesehen werden, die eine genauere Information über die Stellung des Mechanismus liefert.

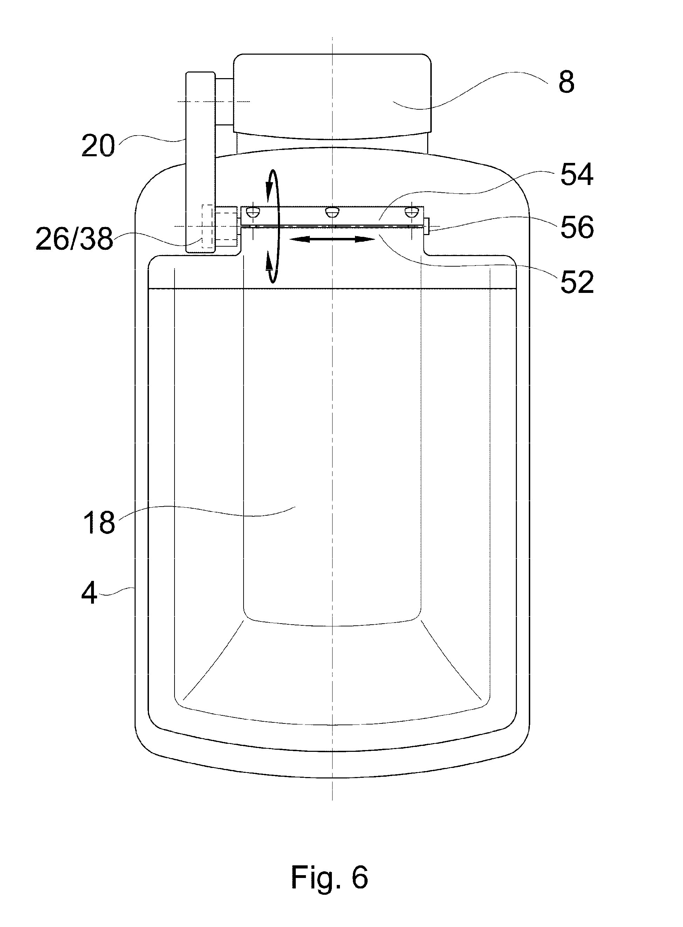

Die Haube kann in einer bevorzugten Ausführungsform über eine Klemmverbindung wie in Figur 6 dargestellt mit dem Kopplungsmechanismus verbunden werden. Ein Teil einer Schelle 52 ist fest in der Haube 18 integriert, der andere Teil der Schelle 54 wird mit dem ersten Teil 52 verschraubt und verklemmt dabei den Bolzen 56, der je nach Ausführungsform fest mit dem Verbindungsglied 26 oder dem zweiten Ritzel 38 verbunden ist. Durch diesen Mechanismus kann die Haube 18 bei der Montage sowohl in transversaler Richtung als auch in ihrer Winkellage zur Liegefläche 4 justiert werden, um so Toleranzen im Gesamtaufbau des Geräts auszugleichen. So kann die Haube 18 im geschlossenen Zustand möglichst genau zur umrandeten Liegefläche 4 positioniert werden, was zum Beispiel für die Abdichtung zwischen den Haubenteilen zweckmäßig ist. Die Figur 6 zeigt außerdem eine bevorzugte Anordnung des Kopplungsmechanismus, indem der Hubarm 20 seitlich an der mittig angeordneten Tragstruktur 8 angeordnet ist.

### Bezugszeichenliste:

- 2: Wärmetherapiegerät
- 4: Liegefläche
- 6: Horizontale Tragstruktur
- 8: Vertikale Tragstruktur
- 10: Hubsäule
- 12: Horizontale Basis
- 14: Rollen
- 16: Strahlungsheizung
- 18: Haube
- 18a: von der Strahlungsheizung abgewandtes Haubenende
- 18b: der Strahlungsheizung zugewandtes Haubenende
- 20: Erste Schwinge oder Hubarm
- 22: Zweite Schwinge
- 24: Verbindungsglied zur vertikalen Tragstruktur 8
- 26: Verbindungsglied zur Haube 18
- 28: Dritte Schwinge
- 30: Feder-Dämpfermechanismus, z. B. Gasdruckfeder
- 32: Anzeige- und Bedieneinheit
- 36: Kettenrad oder Zahnriemenscheibe
- 38: Kettenrad oder Zahnriemenscheibe
- 40: Kette oder Zahnriemen
- 42: Umlenkung
- 44: Spannschraube
- 46: Griff
- 48: Motor
- 50: Endstellungssensoren
- 52, 54: Schellenteile
- 56: Bolzen

## Patentansprüche

1. Wärmetherapiegerät, insbesondere Inkubator, zur Behandlung von Neugeborenen mit einer von oben frei zugänglichen umrandeten Liegefläche (4) zur Aufnahme eines Neugeborenen, einer Haube (18), die zwischen einer die umrandete Liegefläche (4) abdeckenden geschlossenen Stellung und einer die umrandete Liegefläche (4) freigebenden offenen Stellung beweglich ist, und einer an einer Tragstruktur (8) aufgehängten, auf die umrandete Liegefläche (4) gerichteten Strahlungsheizung (16), wobei die Haube (18) an der Tragstruktur (8) so aufgehängt ist, dass sie nach der Bewegung aus der geschlossenen in die geöffnete Stellung mit ihrem der Tragstruktur zugewandten Ende vertikal wenigstens auf Höhe der Strahlungsheizung (16) und horizontal weiter von der Tragstruktur entfernt als diese liegt, so dass die Haube (18) eine Position außerhalb des Strahlungskegels von der Strahlungsheizung auf die Liegefläche einnimmt, **dadurch gekennzeichnet, dass** das der Tragstruktur zugewandte Ende (18b) der Haube (18) durch einen Kopplungsmechanismus auf einer Kreisbahn um einen Drehpunkt an der Tragstruktur geführt wird.

2. Wärmetherapiegerät nach Anspruch 1, **dadurch gekennzeichnet, dass** die Aufhängung der Haube (18) an der Tragstruktur (8) mit einem Kopplungsmechanismus so ausgestaltet ist, dass in der geöffneten Stellung der Haube deren von der Tragstruktur (8) abgewandtes Ende (18a) höher als das ihr zugewandte Ende (18b) liegt.

3. Wärmetherapiegerät nach Anspruch 1 oder 2, **dadurch gekennzeichnet, dass** der Kopplungsmechanismus zwischen Tragstruktur (8) und Haube (18) als viergliedriges Getriebe mit zwei Schwingen (20, 22) ausgeführt ist (Gelenkviereck).

4. Wärmetherapiegerät nach Anspruch 1 oder 2, **dadurch gekennzeichnet, dass** der Kopplungsmechanismus zwischen Tragstruktur (8) und Haube (18) als überbestimmtes fünfgliedriges Getriebe mit drei Schwingen (20, 22, 28) ausgeführt ist.

5. Wärmetherapiegerät nach Anspruch 1 oder 2, **dadurch gekennzeichnet, dass** die Haube (18) durch den Kopplungsmechanismus an der Tragstruktur (8) schwenkbar so aufgehängt ist, dass eine mit der Bewegung der Haube (18) aus der geschlossenen in die geöffnete Stellung einhergehende Winkel- Positionierung der Haube (18) relativ zur Liegefläche (4) durch einen an eine Schwinge (20) gekoppelten Kettentrieb oder Zahnriementrieb mit einem fest mit der Haube (18) verbundenem Kettenrad oder Zahnriemenscheibe (38) und einem fest mit der Tragstruktur (8) verbundenem Kettenrad oder Zahnriemenscheibe (36) bewirkt wird.

6. Wärmetherapiegerät nach Anspruch 5, **dadurch gekennzeichnet, dass** eine Vorrichtung zum Spannen des Ketten- oder Zahnriementriebs vorgesehen ist.

7. Wärmetherapiegerät nach einem der Ansprüche 1 bis 6, **dadurch gekennzeichnet, dass** die Tragstruktur (8) als Zentralsäule ausgebildet ist und dass der Kopplungsmechanismus zwischen Tragstruktur und Haube einseitig an der Tragstruktur montiert ist.

8. Wärmetherapiegerät nach einem der Ansprüche 1 bis 7, **dadurch gekennzeichnet, dass** die Schwinge (20) als allseitig geschlossenes Gehäuse ausgebildet ist, welches die anderen Komponenten des Kopplungsmechanismus umschließt.

9. Wärmetherapiegerät nach einem der Ansprüche 1 bis 8, **dadurch gekennzeichnet, dass** der Kopplungsmechanismus mit einem Feder- und/oder einem Dämpfermechanismus (30) ausgestattet ist.

10. Wärmetherapiegerät nach Anspruch 8 und 9, **dadurch gekennzeichnet, dass** der Feder- und/oder Dämpfermechanismus (30) in die als Gehäuse ausgestaltete Schwinge (20) integriert ist und von dieser umschlossen ist.

11. Wärmetherapiegerät nach Anspruch 8, 9 oder 10, **dadurch gekennzeichnet, dass** der Feder- und/oder Dämpfermechanismus zwischen mindestens einer Schwinge und der Tragstruktur und/oder zwischen zwei Schwingen angeordnet ist.

12. Wärmetherapiegerät nach Anspruch 9, 10 oder 11, **dadurch gekennzeichnet, dass** der Feder- und/oder Dämpfermechanismus (30) so ausgestaltet ist, dass die Haube (18) in die geschlossene Stellung bewegt wird, solange die Haube um weniger als einen Schwellenwert aus der geschlossenen Stellung herausgeschwenkt ist, und sie in die vollständig geöffnete Stellung bewegt wird, sobald die Haube um mehr als den Schwellenwert aus der geschlossenen Stellung heraus geschwenkt ist.

13. Wärmetherapiegerät nach einem der Ansprüche 1 bis 12, **dadurch gekennzeichnet, dass** zur Bewirkung einer Drehbewegung ein Aktuator oder ein Motor (48) an dem Drehpunkt zwischen Kopplungsmechanismus und Tragstruktur zum Öffnen und Schließen der Haube vorgesehen ist.

14. Wärmetherapiegerät nach einem der vorhergehenden Ansprüche, **dadurch gekennzeichnet, dass** die Positionierung der Haube (18) über eine einen Bolzen umschließende Schelle (52, 54) sowohl in transversaler Richtung als auch in ih-rer Winkellage relativ zur Liegefläche (4) einstellbar ist.

15. Wärmetherapiegerät nach einem der vorhergehenden Ansprüche, **dadurch gekennzeichnet, dass** elektrische Endstellungssensoren (50) vorgesehen sind, die die geöffnete und geschlossene Stellung der Haube detektieren.

## Claims

1. A thermotherapy device, in particular an incubator, for treating newborns, having a bordered reclining surface (4) that is freely accessible from above for receiving a newborn, a hood (18) that is movable between a closed position covering the bordered reclining surface (4) and an open position freeing the bordered reclining surface (4), and a radiant heating system (16) that is suspended on a supporting structure (8) and is directed at the bordered reclining surface (4), wherein the hood (18) is suspended on the supporting structure (8) in such a way that after the movement out of the closed position into the open position it lies with its end, which faces the supporting structure, vertically at least at the level of the radiant heating system (16) and horizontally further away from the supporting structure than the heating system (16) so that the hood (18) occupies a position outside the radiation cone from the radiant heating system to the reclining surface, **characterised in that** the end (18b) of the hood (18) that faces the supporting structure is guided by a coupling mechanism on a circular path about a point of rotation on the supporting structure.

2. A thermotherapy device according to claim 1, **characterised in that** the suspension of the hood (18) on the supporting structure (8) is configured with a coupling mechanism in such a way that in the open position of the hood the end (18a) of the hood facing away from the supporting structure (8) lies at a higher level than the end (18b) facing the structure (8).

3. A thermotherapy device according to claim 1 or 2, **characterised in that** the coupling mechanism between the supporting structure (8) and the hood (18) is constructed as a four-membered gear unit with two rocker arms (20, 22) (four-bar linkage).

4. A thermotherapy device according to claim 1 or 2, **characterised in that** the coupling mechanism between the supporting structure (8) and the hood (18) is constructed as an overdetermined five-membered gear unit with three rocker arms (20, 22, 28).

5. A thermotherapy device according to claim 1 or 2, **characterised in that** the hood (18) is pivotably suspended on the supporting structure (8) by means of the coupling mechanism in such a way that angular positioning of the hood (18) relative to the reclining surface (4) associated with the movement of the hood (18) out of the closed position into the open position is brought about by means of a chain drive or toothed belt drive coupled to a rocker arm (20) with a chain wheel or toothed belt pulley (38) fixedly connected to the hood (18) and a chain wheel or toothed belt pulley (36) fixedly connected to the supporting structure (8).

6. A thermotherapy device according to claim 5, **characterised in that** an apparatus is provided for tensioning the chain drive or toothed belt drive.

7. A thermotherapy device according to one of claims 1 to 6, **characterised in that** the supporting structure (8) is formed as a central column, and **in that** the coupling mechanism between the supporting structure and the hood is mounted on the supporting structure on one side.

8. A thermotherapy device according to one of claims 1 to 7, **characterised in that** the rocker arm (20) is formed as a housing that is closed on all sides and encloses the other components of the coupling mechanism.

9. A thermotherapy device according to one of claims 1 to 8, **characterised in that** the coupling mechanism is equipped with a spring mechanism and/or a damper mechanism (30).

10. A thermotherapy device according to claim 8 and 9, **characterised in that** the spring mechanism and/or damper mechanism (30) is integrated into the rocker arm (20) configured as a housing and is enclosed by the rocker arm (20).

11. A thermotherapy device according to claim 8, 9 or 10, **characterised in that** the spring mechanism and/or damper mechanism (30) is arranged between at least one rocker arm and the supporting structure and/or between two rocker arms.

12. A thermotherapy device according to claim 9, 10 or 11, **characterised in that** the spring mechanism and/or damper mechanism (30) is configured in such a way that the hood (18) is moved into the closed position as long as the hood is swung out of the closed position by less than a threshold value, and it is moved into the completely open position as soon as the hood is swung out of the closed position by more than the threshold value.

13. A thermotherapy device according to one of claims 1 to 12, **characterised in that** in order to bring about a rotational movement an actuator or a motor (48) is provided at the point of rotation between the coupling mechanism and the supporting structure for opening and closing the hood.

14. A thermotherapy device according to one of the preceding claims, **characterised in that** the positioning of the hood (18) can be adjusted by way of a clamp (52, 54), enclosing a pin, both in the transverse direction and in terms of its angular position relative to the reclining surface (4).

15. A thermotherapy device according to one of the preceding claims, **characterised in that** electrical end position sensors (50) are provided that detect the open and closed position of the hood.

## Revendications

1. Appareil de thermothérapie, en particulier incubateur, pour le traitement de nouveau-nés, comprenant une surface de couchage encadrée (4) accessible librement par le haut pour la réception d'un nouveau-né, un capot (18) qui peut être déplacé entre une position fermée recouvrant la surface de couchage encadrée (4) et une position ouverte dégageant la surface de couchage encadrée (4), et un chauffage par rayonnement (16) suspendu à une structure portante (8) et dirigé vers la surface de couchage encadrée (4), le capot (18) étant suspendu à la structure portante (8) de telle façon qu'il se situe, après le mouvement de la position fermée dans la position ouverte, avec son extrémité proche de la structure portante (8) verticalement au moins à la hauteur du chauffage par rayonnement (16) et horizontalement à une plus grande distance de la structure portante que celui-ci, de sorte que le capot (18) prend une position extérieure au cône de rayonnement du chauffage par rayonnement sur la surface de couchage, **caractérisé en ce que** l'extrémité (18b) du capot (18) proche de la structure portante est guidée par un mécanisme de couplage sur une trajectoire circulaire autour d'un pivot sur la structure portante.

2. Appareil de thermothérapie selon la revendication 1, **caractérisé en ce que** la suspension du capot (18) à la structure portante (8) avec un mécanisme de couplage est réalisée de telle façon que dans la position ouverte du capot, son extrémité (18a) éloignée de la structure portante (8) se situe plus haut que l'extrémité (18b) proche de celle-ci.

3. Appareil de thermothérapie selon l'une des revendications 1 ou 2, **caractérisé en ce que** le mécanisme de couplage entre la structure portante (8) et le capot (18) est réalisé sous la forme d'un mécanisme à quatre barres avec deux bras oscillants (20, 22) (quadrilatère articulé).

4. Appareil de thermothérapie selon l'une des revendications 1 ou 2, **caractérisé en ce que** le mécanisme de couplage entre la structure portante (8) et le capot (18) est réalisé sous la forme d'un mécanisme à cinq barres surdéterminé avec trois bras oscillants (20, 22, 28).

5. Appareil de thermothérapie selon l'une des revendications 1 ou 2, **caractérisé en ce que** le capot (18) est suspendu à la structure portante (8) par le mécanisme de couplage de telle façon qu'un positionnement angulaire du capot (18) par rapport à la surface de couchage (4) concomitant au déplacement du capot (18) de la position fermée dans la position ouverte soit produit par une transmission à chaîne ou une transmission à courroie crantée couplée à un bras oscillant (20), avec une roue à chaîne ou une poulie de courroie crantée (38) solidaire du capot (18) et une roue à chaîne ou une poulie de courroie crantée (36) solidaire de la structure portante (8).

6. Appareil de thermothérapie selon la revendication 5, **caractérisé en ce qu'**un dispositif pour tendre la transmission à chaîne ou à courroie crantée est prévu.

7. Appareil de thermothérapie selon l'une des revendications 1 à 6, **caractérisé en ce que** la structure portante (8) est réalisée sous la forme d'une colonne centrale et que le mécanisme de couplage entre structure portante et capot est monté d'un côté sur la structure portante.

8. Appareil de thermothérapie selon l'une des revendications 1 à 7, **caractérisé en ce que** le bras oscillant (20) est réalisé sous la forme d'un boîtier fermé de tous côtés qui renferme les autres composants du mécanisme de couplage.

9. Appareil de thermothérapie selon l'une des revendications 1 à 8, **caractérisé en ce que** le mécanisme de couplage est équipé d'un mécanisme à ressort et/ou à amortisseur (30).

10. Appareil de thermothérapie selon les revendications 8 et 9, **caractérisé en ce que** le mécanisme à ressort et/ou à amortisseur (30) est intégré dans le bras oscillant (20) réalisé sous forme de boîtier et renfermé par celui-ci.

11. Appareil de thermothérapie selon l'une des revendications 8, 9 ou 10, **caractérisé en ce que** le mécanisme à ressort et/ou à amortisseur est disposé entre au moins un bras oscillant et la structure portante et/ou entre deux bras oscillants.

12. Appareil de thermothérapie selon l'une des revendications 9, 10 ou 11, **caractérisé en ce que** le mécanisme à ressort et/ou à amortisseur (30) est conçu de telle façon que le capot (18) soit déplacé dans la position fermée aussi longtemps que le capot est pivoté de moins d'une valeur seuil à partir de la position fermée, et soit déplacé dans la position complètement ouverte dès que le capot est pivoté de plus de la valeur seuil à partir de la position fermée.

13. Appareil de thermothérapie selon l'une des revendications 1 à 12, **caractérisé en ce que** pour produire un mouvement de rotation, un actionneur ou un moteur (48) est prévu au pivot entre le mécanisme de couplage et la structure portante pour ouvrir et fermer le capot.

14. Appareil de thermothérapie selon l'une des revendications précédentes, **caractérisé en ce que** le positionnement du capot (18) est réglable aussi bien dans la direction transversale que dans sa position angulaire par rapport à la surface de couchage (4) au moyen d'un collier (52, 54) entourant un boulon.

15. Appareil de thermothérapie selon l'une des revendications précédentes, **caractérisé en ce que** des capteurs de fin course électriques (50) sont prévus pour détecter la position ouverte et fermée du capot.
